# EUROPEAN PATENT APPLICATION

(11) **EP 3 536 795 A2**
(43) Date of publication of application: **11.09.2019**
(21) Application number: 19165355.9
(22) Date of filing: 20.06.2013
(51) Int. Cl.: C12N 15/864, A61K 48/00, C12N 15/86

(54) **WIDESPREAD GENE EXPRESSION**

(30) Priority: 21.06.2012 US 201261662596 P
(62) Divisional of application: 13729777.6
(71) Applicant: Association Institut de Myologie, 75013 Paris (FR)
(72) Inventor: BARKATS, Martine, 94220 Charenton Le Pont (FR); VOIT, Thomas, 91470 Boullay Les Troux (FR)
(74) Representative: Cabinet Becker et Associés

(57) **Abstract**

The present invention relates to improved compositions and methods for delivering and expressing therapeutic genes in mammals. More particularly, the invention stems from the unexpected discovery that a remarkable, massive and widespread therapeutic gene delivery and expression is obtained in mammals when a therapeutic gene is incorporated in a viral vector and administered both into the CSF and into the blood of the mammal. Such a combined administration leads to a surprising and substantial therapeutic benefit in the mammal as compared to administration in one single site, and further enables the use of reduced doses of the virus. The invention may be used in any mammal, including human subjects, and is particularly suited to treat multi-systemic diseases, such as motor neuron or lysosomal disorders, where widespread expression of a therapeutic gene is desirable.

## Description

The present invention relates to improved compositions and methods for delivering and expressing therapeutic genes in mammals. More particularly, the invention stems from the unexpected discovery that a remarkable, massive and widespread therapeutic gene delivery and expression is obtained in mammals when a therapeutic gene is incorporated in a particular class of viral vectors and administered both into the cerebrospinal fluid (CSF) and into the blood of the mammal. As illustrated in a model of spinal muscular atrophy (SMA), such a combined administration leads to a surprising and substantial therapeutic benefit in mammals, as compared to administration in one single site, enabling the use of reduced doses of the vector. Surprisingly, the combined delivery into the CSF and into the blood shows higher efficacy than the same total dose applied either into the CSF or intravenously alone, thereby indicating a supra-additive effect. This supra-additive effect in turn allows to reduce the necessary total dose as compared to either CSF delivery alone or systemic delivery alone, and this in spite of the fact that the vector administered into the CSF will also be distributed to the whole body, and that the vector delivered systemically will also be delivered to the central nervous system. The invention may be used in any mammal, including human subjects, and is particularly suited to treat multi-systemic diseases, such as motor neuron (MN) or lysosomal disorders, where widespread expression of a therapeutic gene is desirable.

### INTRODUCTION

Motor neuron (MN) diseases, such as spinal muscular atrophy (SMA), amyotrophic lateral sclerosis (ALS) or Kennedy's disease, are neurodegenerative disorders characterised by the selective degeneration of MNs in the spinal cord, brainstem and/or motor cortex (Monani 2005; Pasinelli and Brown 2006); (MacLean, Warne et al. 1996). There is no treatment for these diseases, mostly because drug delivery to MNs *via* systemic injections is hindered by the presence of the "blood-brain-barrier" (BBB). This anatomical and physiological barrier is formed by tight junctions between the endothelial cells of the central nervous system (CNS) capillaries and prevents easy passage of molecules between the circulation and the CNS (Scherrmann 2002). The alternative supply of MNs with recombinant proteins injected directly into the CNS parenchyma is also difficult due to the low diffusion of the proteins into the nervous parenchyma, the need for repeated injections (or the implantation of an osmotic pump and the invasiveness of the surgical procedure hampering a potential clinical application.

Failure of the classical pharmacology has led the scientific community to develop new therapeutic strategies based, in particular, on gene transfer technology using viral vectors.

In this regard, the first proposed gene transfer strategies of MN diseases were based on the intrathecal delivery or direct injections of vectors into the spinal cord parenchyma (Davidson, PNAS 2000) (Azzouz, Hottinger et al. 2000). These approaches, however, failed to produce efficient widespread CNS transduction.

Injection of viral vectors into the cerebral ventricles was also used in the aim to transduce the epithelial cells of the choroids plexus and ependyma and to cause secretion of a therapeutic protein in the cerebrospinal fluid (CSF) (Passini and Wolfe 2001). However, diffusion of the recombinant proteins to the nervous tissue was not adequate, and no diffusion of the virus could be observed.

An alternative non-invasive strategy was further developed using retrograde axonal transport of viral vectors to MNs through intramuscular (i.m.) injections. Some viral vectors derived from adenoviruses, adeno-associated viruses (AAV) or equine-anemia viruses pseudotyped with the rabies G glycoprotein (EIAV) may indeed undergo retrograde transport along the MN axons after i.m. injections. Some of these viruses were successfully used to transduce the lower MNs in experimental animals (Finiels et al., 1995; Kaspar et al., 2003; Azzouz et al., 2004). However, the clinical value of this method remains questionable due, in particular, to the large number of injection sites and viral particles that are needed for targeting MNs in pathologies that affect most of the patient's motor units.

In WO2009/043936 and Duque et al (Mol Ther 2009), the inventor has surprisingly discovered that peripheral injection of certain viruses such as AAV9 can cause a substantial transduction of CNS cells *in vivo* showing, for the first time, that it is possible to transfer genes of interest into MNs of neonatal and adult mammals after a single peripheral (e.g., intravenous [i.v.], intraperitoneal [i.p.], or intramuscular [i.m.]) injection.

By continuing their research the inventors have now surprisingly discovered that the efficacy of gene transfer and expression *in vivo* can be substantially improved when the gene is cloned into a particular class of viral vectors and when said vector is administered through a combined CSF/Blood administration protocol. Such a method indeed leads to a surprising and substantial therapeutic benefit (survival, weight gain) in vivo as compared to administration into one single site. The combined administration appears to be supra-additive: the combination is more efficient than the identical dose delivered either into the CSF or systemically. This supra-additive effect in turn enables the use of reduced total doses of the virus in order to achieve the desired effect. The invention therefore provides an improved method for expressing therapeutic genes in vivo and may be used in any mammal, including human subjects. It is particularly suited to treat multisystemic diseases, such as MN or lysosomal disorders.

### SUMMARY OF THE INVENTION

The present invention relates to novel methods for the delivery of therapeutic products in vivo using transferable viral vectors. More specifically, the invention relates to compositions and methods for delivering and expressing therapeutic genes into the nervous system and peripheral tissues of mammalian subjects through combined administration of a transferable viral vector into the CSF and into the blood of the subject.

An object of this invention more specifically relates to a method for expressing a therapeutic gene in a mammal, comprising the combined administration in the CSF and in the blood of said mammal of a transferable viral vector, preferably a transferable AAV vector (tAAV), comprising said gene.

A further object of the invention relates to a method for treating a multi-systemic disease in a mammal by administration of a therapeutic gene effective to treat said disease, wherein the therapeutic gene is comprised in a transferable viral vector, preferably a tAAV vector, and wherein said method comprises the combined administration of the vector into the CSF and into the blood of said mammal.

A further object of the invention resides in a method of treating a CNS disorder, such as a MN disease, in a subject in need thereof comprising administering to said subject a therapeutic gene, wherein the therapeutic gene is comprised in a transferable viral vector, preferably a tAAV vector, and wherein said method comprises the combined administration of the vector into the CSF and into the blood of said mammal, leading to a treatment of the subject.

A further object of the invention resides in a method of increasing survival or weight in a subject having a MN disease by administering to said subject a therapeutic gene, wherein the therapeutic gene is comprised in a transferable viral vector, preferably a tAAV vector, and wherein said method comprises the combined administration of the vector into the CSF and into the blood of said mammal, leading to an increase in the survival or weight of the subject.

A further object of the invention is a transferable viral vector comprising a therapeutic gene for use in the treatment of a multi-systemic disease in a mammalian subject by combined administration of the vector into the CSF and into the blood of the subject.

A further object of the invention is a transferable viral vector comprising a therapeutic gene for use in the treatment of a CNS disorder in a mammalian subject by combined administration of the vector into the CSF and into the blood of a subject in need thereof.

According to a preferred embodiment, the transferable viral vector is a tAAV, such as an AAV9 or an AAV10 vector.

Administration of the viral vector into the CSF of the mammal is preferably performed by intracerebroventricular (i.c.v. or ICV) injection, intrathecal injection, or intracisternal injection, and administration into the blood is preferably performed by parenteral delivery (such as i.v. (or IV) injection, i.m. injection, intra-arterial injection, i.p. injection, subcutaneous injection, intradermal injection, nasal delivery, transdermal delivery (patches for examples), or by enteral delivery (oral or rectal).

As illustrated in the application, the combined administration is, more preferably, a simultaneous administration, although a sequential administration may be performed.

A further object of the invention is a composition or kit comprising two unitary dosages of a transferable viral vector, one unitary dosage adapted for systemic injection, one unitary dosage suitable for injection into the CSF.

A further specific object of the invention resides in a method of treating SMA in a mammal in need thereof, comprising the combined administration into the CSF and blood of said mammal of a transferable viral vector, preferably a tAAV vector, comprising a therapeutic gene, said combined administration leading to expression of therapeutic gene in the nervous system and peripheral tissues or organs and allowing the treatment of SMA.

More preferably, the therapeutic gene for treating SMA is a SMN gene (i.e., any DNA or RNA encoding an SMN protein) or any sequence (such as sequences encoding antisense oligonucleotides) that can modulate alternative splicing, activate the promoter, or increase the stability of SMN protein, thereby causing an increase in SMN levels.

A further object of the invention resides in a method for reducing the dose of therapeutic gene administered to a subject in need of a gene therapy treatment, without reducing the clinical benefit, the method comprising administering said gene with a transferable viral vector into the CSF and into the blood of the subject.

The invention may be used in any mammal, preferably in human subjects.

### LEGEND TO THE FIGURES

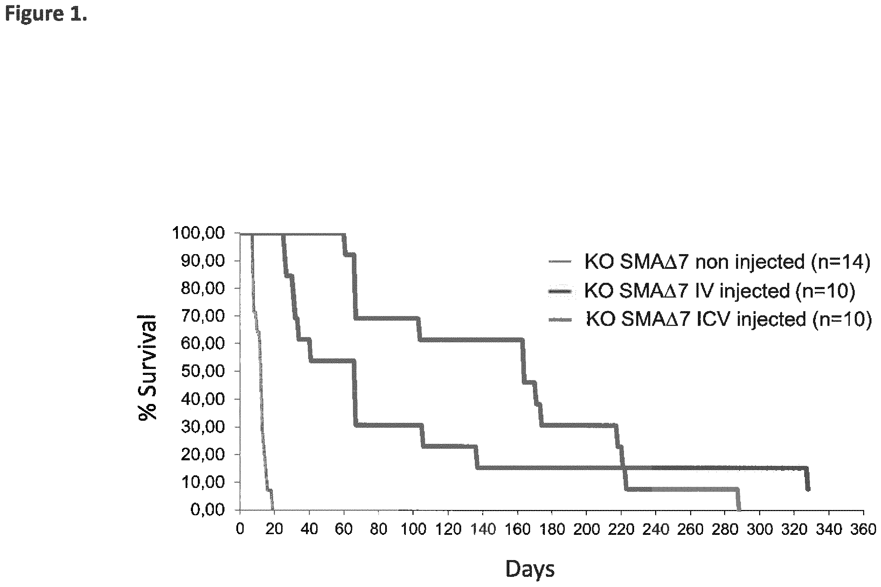

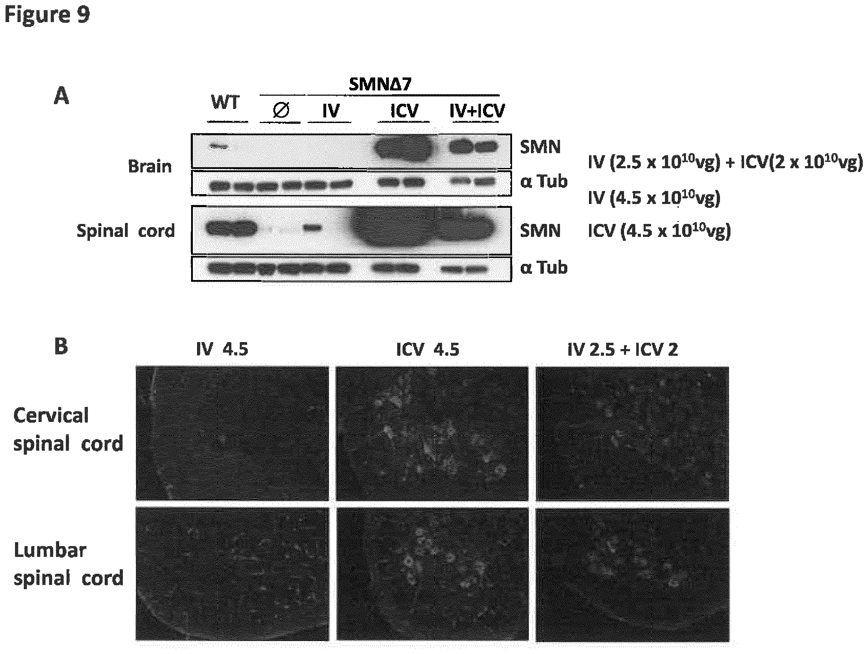

**Figure 1****. Kaplan-Meier survival curve of ICV and IV scAAV9-PGK-SMNopti injected SMNdelta7 mice**
   Both ICV (n=10) and IV (n=10) injected mice (at P0) survived beyond the maximal lifespan of non-injected mice (18 days, n=14). No ICV injected mouse died before the age of 60 days, whereas only 54% of the IV injected mice were alive at this age. The median survival of ICV injected mice was significantly higher than that of IV injected mice (163 days versus 73 days).

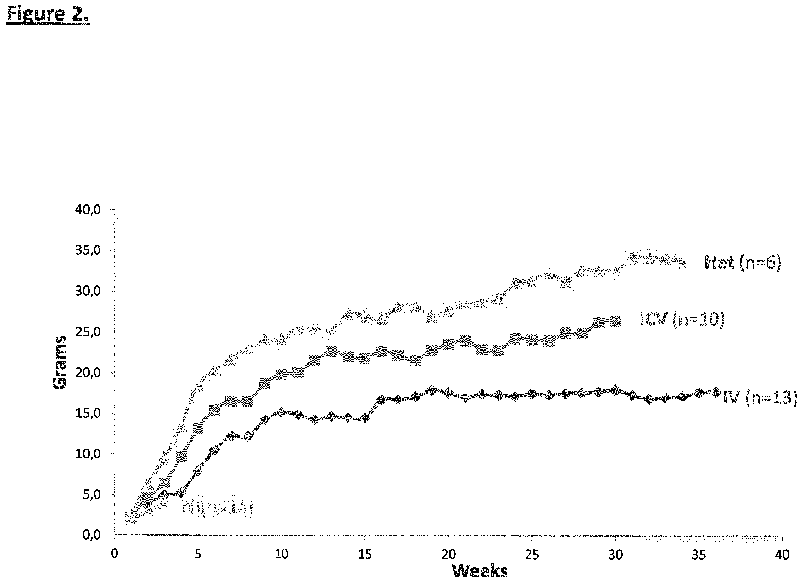

**Figure 2****. Body weight curve of ICV- and IV-injected scAAV9-PGK-SMNopti SMNdelta7 mice**
   The body weight loss phenotype of ICV of SMNdelta7 injected mice (n=10) was improved compared to that of IV injected of SMNdelta7 mice (n=13). The body weight loss phenotype of both IV and ICV injected mice was improved compared to that of non-injected SMNdelta7 mice; For ICV injected mice the improvement was superior to that of IV injected mice (at 30 days of age, ≈17g and ≈25gg for i.v. and i.c.v. injected mice, respectively versus ≈33g g for heterozygous mice and ≈3g for non-treated mice). Ht: control Heterozygous mice; NI: non injected SMNdelta7 mice.
**Figure 3****. Expression of SMN in the central nervous system of SMNdelta7 mice after ICV scAAV9-PGK-SMN injection at P0**
   **(A)** Western-blot and **(B)** immunofluorescence analyses of SMN expression in the brain and spinal cord show higher SMN levels in ICV-injected SMNdelta7 mice (n=3) than in non-injected SMNdelta7 (n=2) and wild-type (n=2) mice.
**Figure 4****. Expression of SMN in peripheral organs of SMNdelta7 mice after ICV scAAV9-PGK-SMN injection at P0**
   Expression of SMN is detected in the heart, the kidney, and the liver of ICV-injected neonatal SMNdelta7 mice (n=3).
**Figure 5****. Kaplan Meier Survival curve of ICV, IV, and Co-ICV/IV scAAV9-PGK-SMNopti injected SMNdelta7 mice**
   There is a trend for a later onset of the disease in the Co-ICV/IV injected mice (n=14) compared to mice injected IV (n=10) or ICV (n=14) alone. 100% of the co-ICV/IV mice are still alive at 50 days of age.
**Figure 6****. Body weight loss phenotype is improved in the Co-ICV/IV injected mice (n=14) compared to mice injected IV (n=10) or ICV (n=14) alone.**
   At 15 weeks, the mean weight body reached 24 g, 21 g, and 16g for the Co-ICV/IV, ICV, and IV, respectively (the weigh body of Heterozygous mice was 26.5g). At 15 weeks, the ANOVA analysis did not show any statistical difference in the weight body between Heterozygous and Co-IV/ICV injected mice (in contrast to ICV or IV injected mice).

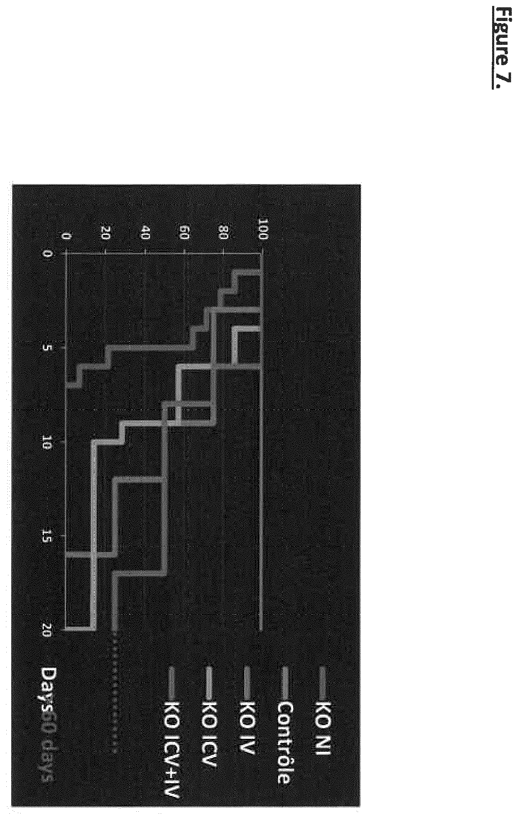

**Figure 7****. Kaplan Meier Survival curve of ICV, IV, and Co-ICV/IV scAAV9-PGK-SMNopti injected hSMN2 mice.**
   The mean survival of the Co-ICV/IV injected mice (≈ 27 days) is superior to that of the IV (≈ 9 days) or (ICV≈ 8 days) injected mice with one of the Co-ICV/IV injected mice surviving up to 170 days.
   Similar differences were found for scAAV10-SMNopti injected mice, with one of the mice aged of 200 days and still surviving at time of this submission.

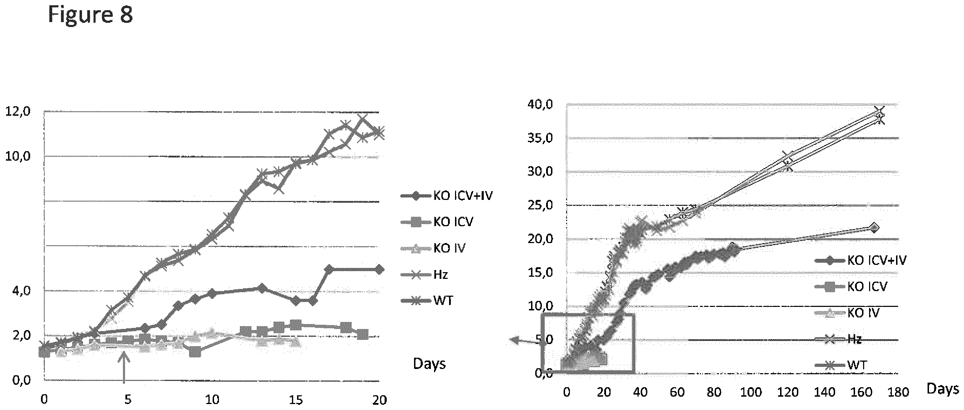

**Figure 8****. Comparison of the body weight phenotype of severe hSMN2 mice (KO) either ICV, IV or Co-ICV/IV injected at P0 with the scAAV9-PGK-SMNopti.**
   The body weight loss phenotype of ICV/IV co-injected KO mice was improved compared to that of IV or ICV injected SMNdelta7 mice (Hz: control heterozygous mice; KO: non injected severe SMA mice; WT: wild type mice). Importantly, one ICV/IV injected mice survived up to 170 days of age
**Figure 9****. Comparison of SMN expression in the CNS of ICV, IV, or Co-IV/ICV scAAV9-PGK-SMN injected SMNdelta7 mice**
   **(A)** Western blot analysis of mouse brain and spinal cord tissues 15 days after injection at P0 with scAAV9-PGK-SMN according to the different delivery routes (n=2 per delivery route), and in non-injected SMNdelta7 and wild-type controls. The SMN levels are reduced in Co-ICV/IV injected mice compared to mice injected with ICV alone. Brain and spinal cord expression of SMN in ICV-injected SMNdelta7 mice. **(B)** Representative sections of spinal cord transversal sections from mice injected at P0 with scAAV9-PGK-SMN according to the different delivery routes, and treated for SMN immunofluorescence 15 days after injection. Again, the number of SMN-positive cells and the intensity of the staining are reduced in Co-ICV/IV injected mice compared to mice injected with ICV alone.

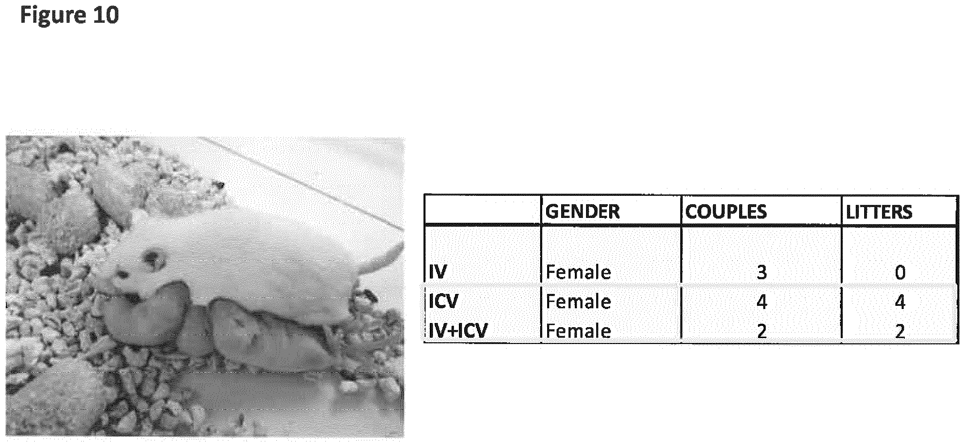

**Figure 10****. Restoration of the reproductive capacity in treated SMA mice.**
   A significant benefit was observed in the SMNdelta7 female mice after ICV or ICV/IV injection in terms of restored reproductive capacity compared to IV injected mice. 100% of the females were fertile in the ICV/IV and ICV treated groups, in contrast to IV treated mice which were not able to reproduce themselves.

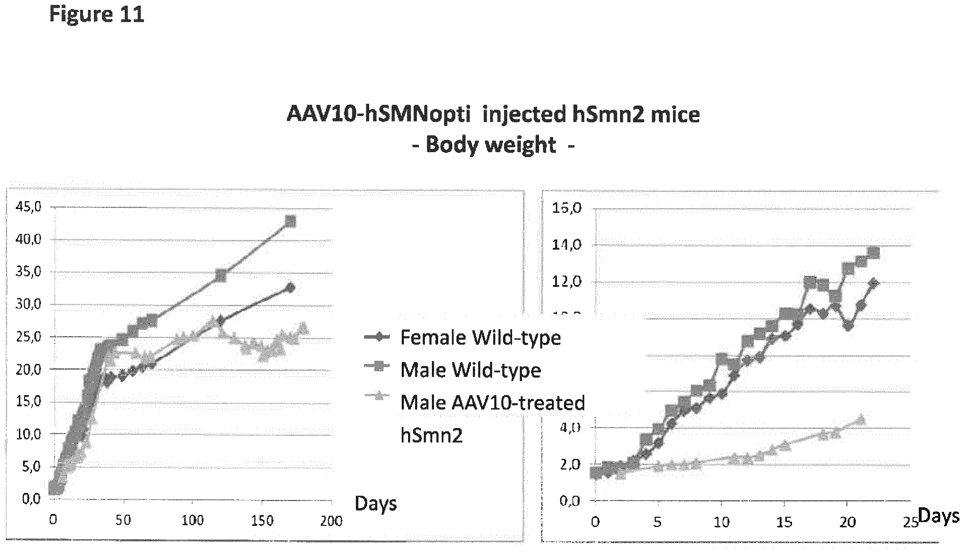

**Figure 11****. Comparison of the survival and body weight phenotype of wild-type mice and Co-ICV/IV AAV10-hSMNopti-injected hSMN2 mice.**
   The body weight loss phenotype and survival of Co-ICV/IV AAV10 injected mice was considerably improved compared to non-treated mice, in particular in two mice aged of 209 and 36 days. Non-treated mice never passed the age of 6 days (median survival of ≈ 3 days).

### DETAILED DESCRIPTION OF THE INVENTION

Widespread gene delivery to both the nervous system and peripheral tissues is an important challenge for the treatment of multi-systemic disorders such as MN diseases (e.g., spinal muscular atrophy (SMA) or amyotrophic lateral sclerosis (ALS)) or lysosomal diseases. Here, we describe a new gene transfer methodology that allows efficient transduction of the nervous system (both central and peripheral) and the peripheral organs after a single combined injection of viral vectors into the blood and the CSF of a mammal. In particular, we injected a tAAV vector intravenously ("i.v." or "IV") and intracerebroventricularly ("i.c.v." or "ICV") in mice and obtained a higher and more substantial clinical benefit than after a single ICV injection in both a severe (hSMN2) and milder (SMNdelta7) mouse model of SMA. An improvement was observed in terms of body weight gain for SMNdelta7 mice, and both survival and body weight gain in both hSMN2 mice. Additional benefit was observed in the milder SMNdelta7 mouse model in terms of restored reproductive capacity compared to IV injected mice. Therapeutic gene expression could be found in MNs and glial cells in the spinal cord, in the dorsal sensory fibers and the dorsal root ganglions, as well as in the heart or liver.

The invention therefore provides a novel improved method useful for treating multi-systemic disorders by virus-mediated gene therapy.

### Transferable Viral vectors

Within the context of the present invention, the term "viral vector" designates any vector which comprises or derives from components of a virus and is suitable to infect mammalian cells, preferably human cells. Typically, a viral vector comprises a recombinant viral genome packaged in a viral capsid or envelope.

A "transferable" viral vector designates a viral vector that is able to diffuse in CNS and non-CNS areas of an organism following CSF injection. In particular, transferable viral vectors are able, upon injection into the CSF to diffuse and transduce cells of the peripheral organs, typically by crossing or by-passing the BBB. The present invention indeed demonstrates that viral vectors can exhibit such a capacity and are able, upon CNS injection, to transduce peripheral organs such as the heart, liver or lung with high efficiency.

Transferable viral vectors of the invention may be derived from various types of viruses. As will be disclosed below, in a most preferred embodiment, viral vectors of the invention are AAV vectors.

The term AAV vector typically designates an AAV viral particle (or virion) comprising at least a nucleic acid molecule (genome) encoding a therapeutic product (e.g., a protein, peptide, or RNA sequence, such as an antisense). As will be discussed below, the AAV may be derived from various serotypes, including combinations of serotypes (i.e., "pseudotyped" AAV) or from various genomes (e.g. single-stranded or self-complementary). In addition, the AAV vector may be replication defective and/or targeted.

Adeno-associated virus (AAV) is a dependent parvovirus, of approximately twenty nanometers in size. Like other parvoviruses, AAV is a single-stranded, non-enveloped DNA virus, having a genome of about 5000 nucleotides in length, containing two open reading frames. The left-hand open reading frame codes for the proteins responsible for replication (Rep), while the right-hand open reading frame encodes the structural proteins of the capsid (Cap). The open reading frames are flanked by two ITR sequences, which serve as the origin of replication of the viral genome. Furthermore, the genome also contains a packaging sequence, allowing packaging of the viral genome into an AAV capsid.

AAV requires co-helper functions (which may be provided e.g. by an adenovirus, or by suitable packaging cells or helper plasmids) to undergo a productive infection in cultured cells. In the absence of such helper functions, the AAV virions essentially enter the cells, migrate to the nucleus as a single-stranded DNA molecule, and integrate into the cells' genome. AAV has a broad host range for infectivity, including human cells, is ubiquitous in humans, and is completely non-pathogenic.

AAV vectors have been designed, produced and used to mediate gene delivery in human subjects, including for therapeutic purposes. Clinical trials are presently ongoing in various countries using AAV vectors. Typically, AAV vectors for use in gene transfer comprise a replication defective AAV genome lacking functional Rep and Cap coding viral sequences. Such replication defective AAV vectors more preferably lack most or all of the Rep and Cap coding sequences, and essentially retain one or two AAV ITR sequences and a packaging sequence. The defective genome is packaged in a viral particle, to form a defective, recombined AAV virus, also termed "AAV vector".

Methods of producing such AAV vectors have been disclosed in the literature, including using packaging cells, auxiliary viruses or plasmids, and/or baculovirus systems (Samulski et al., (1989) J. Virology 63, 3822; Xiao et al., (1998) J. Virology 72, 2224; Inoue et al., (1998) J. Virol. 72, 7024; WO98/22607; WO2005/072364). Methods of producing pseudotyped AAV vectors have also been reported (e.g., WO00/28004), as well as various modifications or formulations of AAV vectors, to reduce their immunogenicity upon in vivo administration (see e.g., WO01/23001; WO00/73316; WO04/112727; WO05/005610; WO99/06562). AAV vectors may be prepared or derived from various serotypes of AAVs, which may be even mixed together or with other types of viruses to produce chimeric (e.g. pseudotyped) AAV viruses.

Preferred examples of tAAVs are human AAV4 vectors, human AAV7 vectors, human AAV9 vectors, human AAV10 vectors, or bovine AAV vectors.

The AAV vector may be derived from a single AAV serotype or comprise sequences or components originating from at least two distinct AAV serotypes (pseudotyped AAV vector), e.g., an AAV vector comprising an AAV genome derived from one AAV serotype (for example AAV9), and a capsid derived at least in part from a distinct AAV serotype.

Specific examples of AAV vectors are:
- vectors comprising an AAV9-derived genome (a nucleic acid molecule comprising an AAV9-derived ITR and an AAV9-derived packaging sequence, operatively linked to a nucleic acid encoding a therapeutic protein, preferably two AAV9-derived ITR flanking an AAV9-derived packaging sequence and a nucleic acid encoding a therapeutic protein) in an AAV9-derived capsid ;
- vectors comprising an AAV10-derived genome in an AAV10-derived capsid;
- vectors comprising an AAV9-derived genome in an AAV10-derived capsid;
- vectors comprising an AAV10-derived genome in an AAV9-derived capsid;
- vectors comprising an AAV2-derived genome in an AAV10-derived capsid;
- vectors comprising an AAV2-derived genome in an AAV9-derived capsid;
- vectors comprising a Bovine AAV-derived genome in a Bovine AAV-derived capsid.

The AAV vector may comprise a modified capsid, including proteins or peptides of non viral origin or structurally modified, to alter the tropism of the vector. As a particular example, the capsid may include a ligand of a particular receptor, or a receptor of a particular ligand, to target the vector towards cell type(s) expressing said receptor or ligand, respectively.

In the AAV vectors used in the present invention, the AAV genome may be either a single stranded nucleic acid or a double stranded, self complementary nucleic acid (McCarty et al., Gene Therapy, 2001), more preferably a self complementary nucleic acid.

A most preferred viral vector for use in the present invention is a scAAV9 vector, a ssAAV9 vector, a scAAV10 vector or a ssAAV10 vector.

As discussed above, the AAV-derived genome comprises a nucleic acid encoding a therapeutic product (e.g., a protein or RNA). Typically, the nucleic acid also comprises regulatory sequences allowing expression and, preferably, secretion of the encoded protein, such as e.g., a promoter, enhancer, polyadenylation signal, internal ribosome entry sites (IRES), sequences encoding protein transduction domains (PTD), and the like. In this regard, the nucleic acid most preferably comprises a promoter region, operably linked to the coding sequence, to cause or improve expression of the therapeutic protein in infected cells. Such a promoter may be ubiquitous, tissue-specific, strong, weak, regulated, chimeric, etc., to allow efficient and suitable production of the therapeutic product in the infected tissue. The promoter may be homologous to the encoded protein, or heterologous, including cellular, viral, fungal, plant or synthetic promoters. Most preferred promoters for use in the present invention shall be functional in nervous cells, particularly in human cells, more preferably in MNs. Examples of such regulated promoters include, without limitation, Tet on/off element-containing promoters, rapamycin-inducible promoters and metallothionein promoters. Examples of promoters specific for the MNs include the promoter of the Calcitonin Gene-Related Peptide (CGRP), a known MN-derived factor or the HB9 promoter. Other promoters functional in MNs include the promoters of Choline Acetyl Transferase (ChAT), Neuron Specific Enolase (NSE), Synapsin, or ubiquitous promoters including Neuron Specific Silencer Elements (NRSE). Examples of ubiquitous promoters include viral promoters, particularly the CMV promoter, the RSV promoter, the SV40 promoter, etc. and cellular promoters such as the PGK (phosphoglycerate kinase) promoter.

In a particular embodiment, the nucleic acid may comprise a leader sequence allowing secretion of the encoded protein. Fusion of the transgene of interest with a sequence encoding a secretion signal peptide (usually located at the N-terminal of secreted polypeptides) will allow the production of the therapeutic protein in a form that can be secreted from the transduced cell. Examples of such signal peptides include the albumin, the β-glucuronidase, the alkaline protease or the fibronectin secretory signal peptides.

According to another specific embodiment, the transgene is fused with PTD sequences, such as the Tat or VP22 sequences, in order to cause or improve secretion of the therapeutic protein from the transduced cells and re-uptake by neighbouring cells.

In a particular embodiment the nucleic acid comprises, operably linked, a promoter and a leader sequence, to allow expression and secretion of the encoded protein.

In a further particular embodiment, the nucleic acid comprises, operably linked, a promoter, a leader sequence and a PTD sequence, to allow expression and secretion of the encoded protein.

In a most preferred embodiment, the promoter is ubiquitous.

Also, viral vectors encoding more than one therapeutic products may be used as well. Such bi- or poly-cistronic viral vectors may for instance encode SMN (or any sequence promoting increased SMN levels), and a sequence encoding a factor promoting another function of therapeutic interest for the treated disease, such as cell survival (e.g. anti-apoptotic or neurotrophic factors), axonal growth or maintenance (e.g. neurotrophins, transforming growth factor or extracellular matrix components), or any sequence conferring a beneficial effect on skeletal or cardiac muscle (e.g. muscle trophic factors, activators of myogenesis).

As discussed above, the AAV vectors may be produced by techniques known per se in the art, as further illustrated in the examples.

### Combined Cerebrospinal fluid and blood administration

The invention is based inter alia on the unexpected discovery that an improved viral-mediated gene expression and disease correction can be obtained when a transferable viral vector is used and is delivered both to the blood and the cerebrospinal fluid (CSF) of a subject. Surprisingly, the results show that such combined administration leads to a substantially increased therapeutic gene expression as compared to the same dose of virus in one single administration site. The invention further shows the combined administration improves the therapeutic benefit in treated subjects, e.g., substantially improves survival and weight gain and restores reproductive capacity.

Within the context of the present invention, blood administration means peripheral administration of the vector directly into the blood stream or into surrounding tissues. Such administration includes, without limitation, systemic injection such as intravenous (i.v.), intramuscular (i.m.), intraperitoneal (i.p.), intra-arterial, subcutaneous or transdermic injections. Most preferred blood administration includes i.v., i.a., or s.c. injection.

Further (although less preferred) routes for blood delivery include intradermal injection, nasal delivery, transdermal delivery (patches for examples), or enteral delivery (oral or rectal).

Blood administration may be accomplished using conventional devices and protocols. For instance, injection may be performed with any available syringe, needle, pump, surgery, etc.

Within the context of the present invention, administration into the CSF means administration of the vector directly into the CSF or into surrounding tissues, allowing release of the viral vector into the CSF. Cerebrospinal fluid fills the cerebral ventricles and surrounds the brain and spinal cord. CSF is essentially produced by the choroid plexuses. Studies suggest that the volume of CSF in adult humans is approximately 150ml, and that about 500ml of CSF are produced each day, consistent with the fact that CSF is continuously produced, circulated and absorbed. The CSF eventually empties into the blood via the arachnoid villi and intracranial vascular sinuses.

By injecting a tAAV vector of the invention into the CSF, the inventors have surprisingly found that the tAAV vector is able to transduce cells in the CNS (including the brain, retina and spinal cord) as well as in the peripheral system (e.g., peripheral nervous system and peripheral organs, including skeletal muscle and heart). Injecting tAAV into the CSF may even lead to a stronger gene expression and clinical benefit than peripheral administration alone.

CSF administration according to the invention specifically includes i.c.v. injection, intrathecal (i.t.) injection, or intracisternal (i.c) injection. Preferred CSF administration includes ICV or IT injection. Most preferred administration comprises injection in at least one cerebral lateral ventricle.

In this regard, viral vectors of the invention may be delivered directly to the CSF by injection into the cerebroventricular region (e.g., into one or both of the cerebral lateral ventricles which are filled with CSF) with any suitable device such as a needle, syringe, cannula, or catheter. Such administration may be performed using neurosurgical techniques known per in the art (Davidson et al., PNAS 97:3428-3432, 2000). For i.c.v. injection, the total volume of injected viral vector solution is typically between 0.1 to 5 ml.

Intrathecal injection may also be accomplished using techniques known per se in the art. Upon i.t. injection in patients, AAV vectors could be administered intrathecally by a lumbar puncture, a safe procedure routinely performed at the bedside which allows release of the viral particles into the surrounding CSF (Beutler AS, Curr Opin Mol Ther. 2005 Oct;7(5):431-9)

The administration of a viral vector specifically to a particular region of the CNS may be done by stereotaxic microinjection. To that purpose, one or several images of the brain can be generated using e.g., high resolution MRI, and the resulting images can be transferred to a computer that directs stereotaxic injection. If necessary, specific structures within the human brain may be identified using common general knowledge (see e.g., The Human Brain: Surface, Three-Dimensional Sectional Anatomy With MRI, and Blood Supply, 2nd ed., eds. Deuteron et al., Springer Vela, 1999).

As indicated above, the invention resides in a method of viral-mediated gene therapy based on a combined administration into CSF and blood.

Within the context of the invention the term *"combined"* administration is meant to designate the administration of the vector in the two compartments within a time frame sufficiently short to generate a combined effect in vivo without substantial immune interference. More preferably, a "combined" administration comprises the administration into the CSF and into the blood of said mammal within less than 72 hours from each other, preferably within less than 48 hours, more preferably within less than 24 hours, further more preferably within less than 1 hour from each other. The combined administration may be performed in the CSF first and subsequently in the blood, or inversely.

In a preferred embodiment, the combined administration is a substantially simultaneous injection, i.e., both injections are performed essentially at the same time or one right after the other.

The doses and CSF/blood ratio of viral vectors may be adapted by the skilled artisan, e.g., depending on the disease condition, the subject, and the treatment schedule. The viral vectors are typically administered in a "therapeutically-effective" amount, i.e., an amount that is sufficient to alleviate (e.g., decrease, reduce, block, or correct) at least one of the symptoms associated with the disease state, or to provide improvement in the condition of the subject. The effective dose may be a dose sufficient to increase survival. Typically, at total dose of from 10⁹ to 10¹⁶ viral vectors (i.e., particles or viral genomes) is used, preferably from about 10¹⁰ to 10¹⁵, which is split in two unitary dosages for CSF/blood administration, respectively. An illustrative dose is comprised between 5x10¹⁰ to 5x10¹⁴/kg.

In this regard, the relative amount of viral vector administered in the CSF and in the blood may be adjusted to provide optimal clinical benefit. In the regard, the inventors have shown that the ratio: dose administered in CSF/dose administered in the blood shall preferably be comprised between 0.2 and 5, even more preferably between 0.2 and 3. Specific examples of preferred ratios are 0.2, 0.4, 0.6, 0.8, 1.0 or 1.25.

The treatment may consist of a single combined administration, or may be repeated. If administration is to be repeated, the subsequent administrations may be either single (i.e., in only one site selected from CSF or blood) or combined. Also, where repeated administrations are performed, distinct virus serotypes are preferably used, in alternance. As an example, the first combined administration may be performed using an AAV9 vector and the second administration using an AAV10 vector, or inversely.

The viral vector may be administered in any suitable form, either as a liquid solution or suspension, as a solid form suitable for solution or suspension in liquid prior to injection, as a gel or as an emulsion, or spray. The viral vectors are typically formulated with any appropriate and pharmaceutically acceptable excipient, carrier, adjuvant, diluent, etc. For injection, the excipient may be a liquid, isotonic solution, buffer, such as a sterile and pyrogen-free water or a sterile and pyrogen-free phosphate-buffered saline solution.

In a particular embodiment, the invention resides in a method for treating a multi-systemic disease in a mammal by administration of a therapeutic gene effective to treat said disease, wherein the therapeutic gene is comprised in an AAV9 or AAV10 vector and wherein said method comprises the combined i.c.v. and i.v. administration of the vector at a dose ratio i.c.v./i.v. comprised between 0.3 and 3.

As indicated above, the invention may be used for treating nervous system diseases, preferably CNS or multi-systemic nervous diseases. The treatment may be used to alleviate the pathology, in particular to improve survival, weight, reproductive capacity, the motor function, the number of MNs or the muscle size.

### Kits

The invention also relates to compositions and kits. In this regard, a further object of the invention is a composition or kit comprising two unitary dosages of a transferable viral vector comprising a therapeutic gene, one unitary dosage adapted for systemic injection, one unitary dosage suitable for injection into the CSF. The i.c.v./systemic dose ratio is preferably comprised between 0.3 and 3, even more preferably selected from 0.2, 0.4, 0.6, 0.8, 1.0, or 1.25.

The kits of the invention generally comprise one or more separate containers comprising each the unitary CSF and blood dosage. The kits may also comprise a set of instructions, generally written instructions, relating to the use of the vectors for any of the methods described herein. The kits may also include devices or components suitable for administration of the unitary dosages, such as an ampoule, syringe, needle, or the like.

### Motor Neuron disorder

The invention may be used to treat a variety of disorders through the massive and widespread delivery of a therapeutic product. The therapeutic product may be any protein, peptide or RNA that may alleviate or reduce the cause or symptoms of the disease or that otherwise confers a benefit to a subject. Examples of therapeutic proteins include growth factors, cytokines, hormones, neurotransmitters, enzymes, anti-apoptotic factors, angiogenic factors, and any protein known to be mutated in pathological disorders such as the "survival of motor neuron" protein (SMN). Examples of therapeutic RNA include antisense RNA or RNAi targeting messenger RNAs coding for proteins having a therapeutic interest in any of the diseases mentioned herein below. For example, an RNAi targeting the superoxide dismutase enzyme may be coded by an AAV vector as defined above, in view of the treatment of ALS.

A list of preferred candidate therapeutic products is provided below:
SMN (SMA)
IGHMBP2 (SMA-RD)
VAPB (SMA or ALS)
Antisense oligonucleotide sequences targeting pre-mRNA splicing sites of SMN2 (SMA)
Antisense oligonucleotide sequences targeting pre-mRNA splicing sites of SOD1 (ALS) or ATX (Spinocerebellar ataxia) or UBQLN2 (ALS) or ATAXIN2 (ALS) or C9orf72 (ALS) or TARDBP (ALS) or TDP-43, FUS, (ALS and FTD)
shRNA (microRNA or siRNA) targeting mRNA of SOD1 (ALS) or ATX (Spinocerebellar ataxia) or UBQLN2 (ALS) or ATAXIN2 (ALS) or C9orf72 (ALS or frontotemporal lobar degeneration |FTD]) or TARDBP (ALS) or TDP-43 (ALS and FTD) or FUS, (ALS and FTD) or CHMP2B (ALS)
ATX (Spinocerebellar ataxia) or UBQLN2 (ALS) or ATAXIN2 (ALS) or C9orf72 (ALS or frontotemporal lobar degeneration |FTD]) or TARDBP (ALS) or TDP-43 or FUS, (ALS and FTD) or CHMP2B (ALS)
MECP2 or shRNA targeting MECP2 (Rett syndrome)
HEXB (Sandhoff disease)

Depending on the therapeutic product, the invention can be used to treat various diseases, including any disease which may be treated or prevented by expressing therapeutic proteins into, or suppressing toxic proteins from, the nervous tissue. Such diseases include central or peripheral nervous disorders, preferably selected from neurodegenerative diseases, neuromuscular diseases, trauma, bone marrow injuries, pain (including neuropathic pain), cancers of the nervous system, demyelinating diseases, autoimmune diseases of the nervous system, neurotoxic syndromes, or multi-systemic diseases such as lysosomal or peroxisomal diseases,

Specific examples of diseases include Alzheimer's Disease, Parkinson's Disease, Huntington's Disease, Tourette Syndrome, schizophrenia, Sly disease, Hunter's disease, dementia, paranoia, obsessive compulsive disorder, mental retardation, amyotrophic lateral sclerosis, spinal muscular atrophy, Charcot-Marie Tooth disease, spinocerebellar ataxia, spastic paraplegia, Kennedy's disease, glioblastoma, neuroblastoma, autism, Gaucher's disease, Hurler's disease, Krabbe's disease and altered behaviors (e. g., disorders in sleeping, perception or cognition).

The invention may be used in any mammalian, particularly in human subjects, including adults, for preventive or curative treatment.

### DISCLOSED ITEMS

Item 1. A method for expressing a therapeutic gene in a mammal, comprising the combined administration into the cerebrospinal fluid and into the blood of said mammal of a transferable viral vector comprising said gene.
Item 2. A method for treating a multi-systemic disease in a mammal by administration of a therapeutic gene effective to treat said disease, wherein the therapeutic gene is comprised in a transferable viral vector and wherein said method comprises the combined administration of the vector into the cerebrospinal fluid and into the blood of said mammal.
Item 3. The method of item 1 or 2, wherein the transferable viral vector is a transferable AAV (tAAV) vector.
Item 4. The method of item 3, wherein the tAAV vector is selected from an AAV4 vector, an AAV7 vector, AAV9 vector, an AAV10 vector, or a bovine AAV vector.
Item 5. The method of anyone of items 1 to 4, wherein the genome of the tAAV vector is single- or double-stranded.
Item 6. The method of any one of items 1 to 5, wherein the tAAV vector comprises a replication defective AAV genome lacking functional Rep and Cap coding viral sequences.
Item 7. The method of any one of items 1 to 6, wherein the tAAV vector is a ssAAV9 vector, an scAAV9 vector, or an AAV10 vector.
Item 8. The method of item 1 or 2, wherein administering the transferable viral vector in the cerebrospinal fluid (CSF) of the mammal is performed by i.c.v. injection (ICV), intrathecal injection, or intra-cisterna magna injection.
Item 9. The method of item 8, which comprises administering the vector at least into one cerebral lateral ventricle.
Item 10. The method of item 1 or 2, wherein administering the transferable viral vector in the blood of the mammal is performed by intravenous injection (IV), intramuscular injection, intraarterial injection, intraperitoneal injection, or subcutaneous injection.
Item 11. The method of item 10, which comprises an intravenous injection of the vector.
Item 12. The method of item 1 or 2, which comprises the combined ICV and IV injection of the vector.
Item 13. The method of item 1 or 2, wherein the combined administration comprises the administration in the CSF and in the blood of said mammal within less than 72 hours from each other, preferably within less than 48 hours, more preferably within less than 24 hours, further more preferably within less than 1 hour from each other.
Item 14. The method of item 12, wherein the combined administration is a substantially simultaneous injection.
Item 15. The method of item 1 or 2, wherein the ratio: dose administered in the CSF/dose administered in the blood is comprised between 0.2 and 5, preferably between 0.2 and 1.5.
Item 16. The method of item 13, wherein said ratio is 0.4, 0.6, 0.8, 1.0, or 1.25.
Item 17. The method of any one of the preceding items, wherein the therapeutic gene encodes a therapeutic RNA or protein selected from growth factors, cytokines, hormones, neurotransmitters, enzymes, anti-apoptotic factors, angiogenic factors, any protein known to be mutated in pathological disorders such as the "survival of motor neuron" protein (SMN).
Item 18. The method of any one of items 2 to 17, wherein the multi-systemic disease is selected from neurodegenerative diseases, neuromuscular diseases, pain, lysosomal diseases, trauma, bone marrow diseases, cancers of the nervous system, demyelinating diseases, autoimmune diseases of the nervous system, neurotoxic syndromes, sleeping disorders.
Item 19. The method of any one of the preceding items, which comprises a single combined CSF-Blood administration.
Item 20. A method of treating SMA in a mammal in need thereof, comprising the combined administration into the cerebrospinal fluid and blood of said mammal of a tAAV vector comprising a SMN gene, said combined administration leading to expression of SMN protein in nervous system and peripheral tissues and organs and allowing the treatment of SMA.
Item 21. A kit comprising two unitary dosages of a tAAV vector comprising a therapeutic gene, one unitary dosage being adapted for systemic injection, and one unitary dosage being adapted for injection into the CSF.
Further aspects and advantages of the present inventions will be disclosed in the following experimental section, which shall be considered as illustrative only, and not limiting the scope of this application.

### EXAMPLES

### Materials and Methods

### Animals

SMNdelta7 mice (considered as a model of SMA type I) were purchased from the Jackson Laboratory (SMN2+/+, SMNdelta7+/+, Smn-/-, JACKSON no. SN 5025). These mice are triple mutant invalidated for the endogenous murine Smn gene by targeted mutation of Exon 2 and harboring two transgenic alleles (the human SMN2 cDNA [lacking exon 7] and the entire human SMN2 gene) (Le T.T. Hum Mol Genet 2005). Wild-type mice (WT) corresponded to [SMN2+/+, SMNdelta7+/+, Smn+/+] littermates, and heterozygous (Ht) to [SMN2+/+, SMNdelta7+/+, Smn+/-] littermates. The mice were bred to generate self-sustaining colonies and maintained under controlled conditions (22 ± 1°C, 60 ± 10% relative humidity, 12 h/12 h light/dark cycle, food and water ad libitum). All animal experiments were carried out according to the European guidelines for the care and use of experimental animals.

### Production of scAAV vectors

AAV2 plasmids expressing either the GFP transgene under control of the CMV promoter or a codon-optimized sequence of hSMN1 (SMNopti) under control of the phosphoglycerate kinase (PGK) promoter were used to generate the corresponding pseudotyped AAV9 vectors as previously described (Duqué et al., Mol Ther 2009; Dominguez et al., Hum Mol Genet 2011) with slight modifications. Briefly, AAV9-SMN was produced by helper virus-free three-plasmid transfection in HEK293 cells, using (i) the adenovirus helper plasmid, (ii) the AAV packaging plasmid encoding the rep2 and cap9 genes (p5E18-VD2/9) and the AAV2 plasmid expressing GFP or SMNopti. The recombinant vectors were purified by ultracentrifugation on an iodixanol density gradient. Viral preparations were desalted and concentrated using Amicon Ultra-Ultra cell 100 K filters units (Millipore). Aliquots were stored at -80°C until use. Vector titers were determined by real-time PCR and expressed as viral genomes per milliliter (vg/ml).

### In vivo AAV injection

### Neonates:

The i.v. injections were performed using a Hamilton syringe with a 33G needle in the blood flow way of the temporal vein near the eyes. The i.c.v. injections were performed using a Hamilton syringe with a 33G needle, 1mm anterior, 1mm lateral to the lambda and 2mm deep. Both IV and ICV injections were validated using a blue dye solution.

Neonatal SMNdelta7 mice or control wild-type littermates were injected with the viral solutions on the day of birth (P0). For single i.c.v injections, AAV9-GFP (1.7 × 10e10 vg per mouse, 7 µl) or AAV9-SMNopti (4.5 × 10e10 vg per mouse, 7 µl) were unilaterally injected into the right lateral ventricle. For single i.v. injections, the same concentrations of vectors were injected into the temporal vein in a volume of 70µl (in PBS). For i.v. and i.c.v. combined injections, 2.5x10e10vg (30µl) of AAV9-SMNopti was injected into the temporal vein and 2x10e10vg (3 µl) of AAV9-SMNopti was unilaterally injected into the right lateral ventricle.

Neonatal Smn-/-SMN2+/+ mice and wild-type control littermates were injected at birth using a similar procedure. For single i.c.v injections of AAV9-GFP and AAV9-SMNopti, the vector doses and volumes were the same as for neonatal SMNdelta7 mice. For i.v. injections, AAV9-GFP (1.2 × 10e11 vg per mouse) or AAV9-SMNopti (3.4 × 10e11 vg per mouse), were injected into the temporal vein in a volume of 50µl (in PBS). For i.v. and i.c.v. combined injections, 4.5x10e10vg (50µl) of AAV9-SMNopti was injected into the temporal vein and 4.5x10e10vg (7 µl) of AAV9-SMNopti was unilaterally injected into the left lateral ventricle.

*Adults.* One month aged wild-type mice (n=4) were injected with scAAV9-GFP (4.8 × 10e10 vg, 20 µl) into the right lateral ventricle. Injections were performed under intra peritoneal anesthesia (kétamine (100mg/Kg), xylazine (10mg/kg)). Stereotactic injections were performed directly in the left brain ventricule (0.34 mm posterior, 1.0 mm lateral to the bregma and 1.8 mm deep from the top of the brain).

### Histology

The mice were euthanized 15 days and 1month post injection for SMN and GFP expression analyses, respectively. Mice were anesthetized (10 mg/kg xylazine, 100 mg/kg ketamine) and perfused intracardially with 0.1 mol/l PBS, followed by 4% paraformaldehyde (PFA) in PBS. Muscles were frozen after PBS perfusion only (except for P15 neonates). Tissues were removed and postfixed by incubation for 24 hours in the same PBS-4% PFA solution. They were then incubated at least 24 hours at 4 °C in a PBS-15% sucrose solution for brains and non-nervous organs and a PBS-30% sucrose solution for spinal cords, before being frozen in cold isopentane (-50 °C). Serial sections were cut on a cryostat (16 µm sections for brain; 14 µm for spinal cord and heart; 12 µm for spleen and kidney; 10µm for liver; 8µm sections for muscles) and were stored at -80 °C for further analysis.

### Immunostaining analysis

For immunohistochemical detection of GFP in tissues from the AAV9-GFP injected mice, sections were first washed in PBS Triton X-100 0.1% and then incubated for 20 min in a solution of PBS buffer with hydrogen peroxide 1%, methanol 20% and Triton X-100 0.1% (or an hydrogen peroxide solution [Peroxidase Blocking Solution; Dako, Trappes, France]) to block the endogenous peroxidase activity. Sections were blocked for 1 hour at room temperature in PBS buffer with goat serum (Dako) 4%, bovine serum albumin (Sigma) 4% and Triton X-100 0.1%. Sections were then incubated overnight with a rabbit polyclonal anti-GFP (1/5000; Abcam). After rinsing, sections were incubated with a horseradish peroxidise (HRP) biotinylated anti-rabbit antibody (1/250; Vectastain, Vector Laboratories) for 2 hours and then with an avidin-biotin complex (Vectastain Elite ABC kit, Vector Laboratories) for 30 min. After rinsing with PBS Triton X-100 0.1%, diaminobenzidine (DAB) staining was revealed with the 3.3'-DAB substrate kit for peroxidase from Vector Laboratories. Reaction was stopped with water, and the sections were dehydrated in alcohol and xylene before being mounted with Eukitt.

For double NeuN/GFP-immunofluorescence analysis, the cryosections were blocked by incubation for 1 hour with 5% of bovin serum albumin (Sigma), 3% of donkey serum (Millipore) and 0.4% Triton X-100 in PBS and then overnight with rabbit polyclonal anti-GFP anti-bodies (1:2000; Abcam). Sections were washed in PBS and then incubated for 1 hour at room temperature with Alexa 488 conjugated donkey anti-rabbit IgG (1:500; Invitrogen). Sections were washed in PBS, and blocked following the kit MOM protocole. Sections were then blocked overnight with mouse anti-NeuN antibodies (1/300; MAB377, Chemicon International). Sections were washed in PBS and then incubated for 1 hour at room temperature with Alexa 594 conjugated donkey anti-mouse IgG (1:500; Invitrogen). Sections were washed in PBS, mounted with fluoromount-G (Calbiochem), and stored at 4°C before observation by confocal microscopy (Axio Imager Z1, Zeiss) and AxioVision 4.7 software (Zeiss).

### Western Blot analysis

Protein extracts were prepared from the brain, spinal cord, heart, liver, kidney and spleen from 14 days old untreated SMNdelta7 mice (*n*=3), AAV9-SMNopti treated SMNdelta7 mice (*n*=3) and WT mice (*n*=3), and from 1 and 2 month-old WT mice injected with AAV9-GFP injected (n=3) or non injected (*n*=3). The tissue extracts were prepared using the Qproteome FFPE tissue kit according to the manufacturer's protocol (Qiagen). Muscles were lysed in RIPAE buffer (150 mm NaCl, 50 mm Tris-HCl, 0.5% sodium deoxycholate, 1% NP40, 1% SDS) supplied with a protease inhibitors cocktail (Complete Mini, Roche Diagnostics). Eighty five or seventy (for the liver) micrograms of total proteins were run on SDS 10% polyacrylamide gels and transferred to Immobilon-p membranes (Millipore). The membranes were incubated successively with a rabbit anti-GFP antibody (1:2000; AbCam) or with a mouse anti-SMN antibody (1:1000; BD transduction) and a mouse anti-α Tubulin (1:10000; Sigma) diluted in the TBST blocking buffer (Tris-buffered saline containing 0.2% Tween 20 supplemented with 5% nonfat dry milk). After four washes in TBST buffer, the membranes were incubated with a horseradish peroxydase-conjugated anti-mouse or anti-rabbit antibody (GE Healthcare, 1:10 000) diluted in the blocking buffer. The membranes were further processed using the chemiluminescence Super Signal Ultra reagent (Pierce).

### Protocol for Combined AAV administration into SMA mice SMNdelta7

**Vector:** AAV9-PGK-SMN (CsCl Gradient purification)
Titer: 6,7x10e12vg/ml
**SMA mouse:** SMNdelta7 (mild SMA, SMA type 2 model)
median survival 13.6 days (maximal life expectancy 18 days)

### Doses

ICV (n=10): 4.5x10e10vg per mouse
IV (n=10): 4.5x10e10vg per mouse
ICV+IV (n=9): 2.5x10e10vg IV + 2x10e10vg ICV

### Protocol for Combined AAV administration into SMA mice smn-/-

**Vector:** AAV9-PGK-SMN (CsCl Gradient purification)
Titer: 6.7x10e12vg/ml
**SMA mouse model: [Smn-/-; hSmn1 +/+] (« KO »)** (severe SMA, SMA type 1 model)
median survival 3.6 days (max lifespan 6 days)

### Doses

KO ICV (n=7): 4,7.10¹⁰ vg per mouse (7 µl)
KO IV (n=4): 3,35.10¹¹vg per mouse (50µl)
KO ICV+IV (n=4): (4,7.10¹⁰ vg ICV + 4,7.10¹⁰ vg IV), total 9,4.10¹⁰ vg per mouse

### Results

### • Survival and phenotype of i.c.v. versus i.v. AAV9-SMNopti injected SMNΔ7 mice.

We used an optimized scAAV9 vector which expresses a codon-optimized version of the human *SMN1* gene under control of the human phosphoglycerate kinase (PGK) promoter, and includes a chimeric intron between the PGK promoter and the *SMN1* cDNA in order to improve translation efficiency and transgene expression (AAV9-SMN) (Dominguez, Hum. Mol. Genet. 2011).

Thirty-five SMNΔ7 mice were injected at P0 with the AAV9-SMNopti vector (4.5x10¹⁰vg per mouse, 3x10¹³vg vg/kg) into either the temporal vein (70µl, n=13), the brain lateral ventricles (7µl, n=13) or into both sites (2.5x10¹⁰vg i.v. in 30µl; 2.5x10¹⁰vg i.c.v. in 3µl, n=9). Controls were non-injected SMNΔ7 (n=14) or heterozygous (n=14) littermate mice.

The phenotypic characterization of the animals involved daily analysis of survival, body weight, peripheral necrosis, overall general appearance, and monitoring of motor activity at 70 days of age. All AAV9-SMN injected SMNΔ7 mice survived beyond the maximal lifespan of non-injected mice (18 days). However, the therapeutic advantage conferred by i.c.v. AAV9-SMNopti surpassed that of the i.v. treatment (**Fig. 1**). Indeed, the same dose of AAV9-SMNopti administered into the brain ventricles delayed the onset of mortality by 35 days compared to i.v. (60 versus 25 days). The median survival increased significantly when the mice were i.c.v. injected with AAV9-SMNopti as compared to i.v. injected mice (from 11.5 days for non-injected mice to 163 and 73 days for i.c.v. and i.v. respectively). Our longest lived mouse in the i.v. injected group is 327 days old to date, whereas our last i.c.v. injected mouse died at 286 days of age.

### • Improvement of the body weight loss phenotype of i.c.v. versus i.v. AAV9-SMNopti injected SMNΔ7 mice.

Both i.v. and i.c.v. AAV9-SMN injected mice gained weight throughout the study period and their body weight was significantly higher than that of non-injected SMNΔ7 mice at 14 days (end-stage of the disease) (mean body weight of 4.7g and 5.5g for i.v. and i.c.v. respectively, versus 3.1g for non-injected mice) (**Fig. 2**). At 6 weeks following injection, the mean body weight of i.c.v. injected mice was nearly that of heterozygous mice, in contrast to i.v. injected mice which appeared significantly smaller (the body weight of i.v. and i.c.v. injected mice was 11.0±1.3 and 16.3±1.2, respectively, versus 18.3±0.6 for heterozygous mice).

### • Expression of SMN in the central nervous system of SMNdelta7 mice after ICV scAAV9-PGK-SMN injection at P0

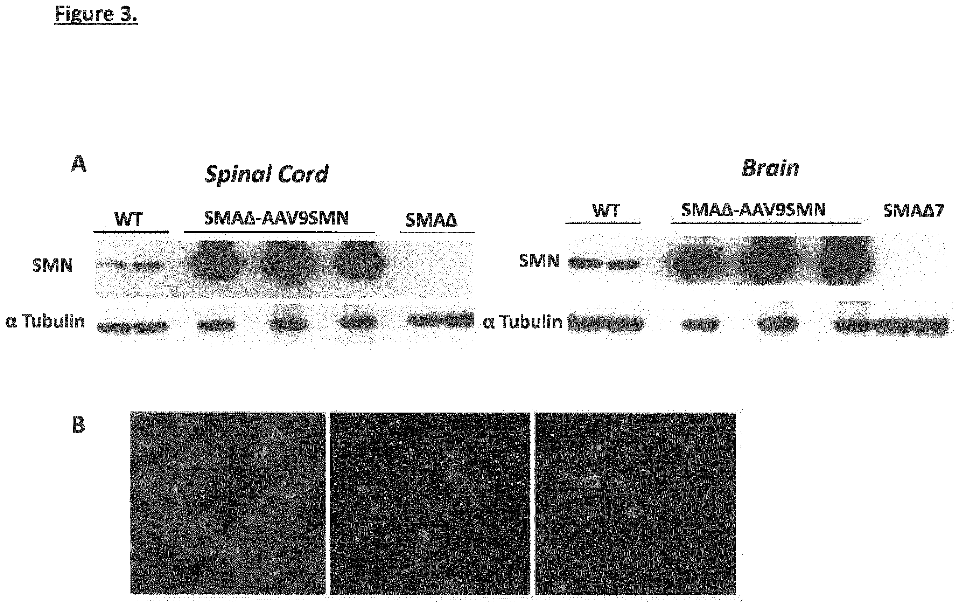

Western blot analysis demonstrated that brain and spinal cord expression of SMN in ICV-injected SMNdelta7 mice (n=3) was significantly higher than in non-injected SMNdelta7 (n=2) and even wild-type animals (n=2) (**Fig.3A**). The increase of SMN expression levels in the CNS of i.c.v. injected mice was superior to that previously reported following i.v. injection of the same concentration of vector (at P0, in the temporal vein) (Duque et al., Mol. Ther. 2009). The high CNS expression induced by i.c.v. injection was confirmed by the presence of a number of GFP positive astroglial and neuronal cells in the brain and the spinal cord of mice injected i.c.v. with a GFP encoding scAAV9 vector (**Fig.3B**). This suggested that SMN expression in the CNS could be sufficient to rescue SMNdelta7 mice from SMA pathology.

### • Expression of SMN in peripheral organs of SMNdelta7 mice after ICV scAAV9-PGK-SMN injection at P0

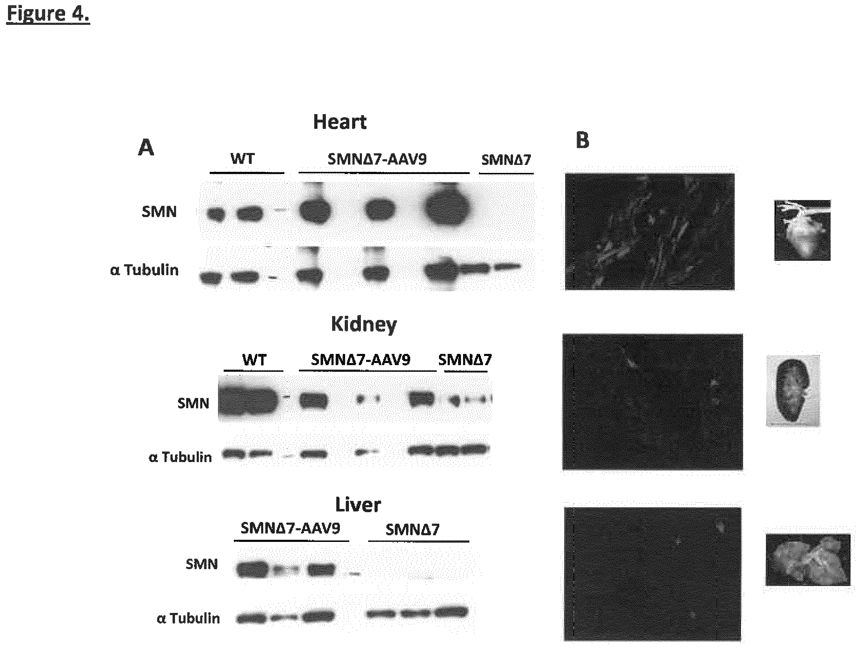

Unexpectedly, expression of SMN was detected in peripheral org ans following i.c.v. delivery of scAAV9-PGK-SMN at P0. In particular, the SMN protein was found in the heart the kidney and the liver of ICV-injected SMNdelta7 mice (n=3) (**Fig. 4A**). These results were confirmed by GFP immunofluorescence analysis in mice injected in the same conditions with a GFP-encoding scAAV9 vector (**Fig4B**).

### • Survival percentage after ICV / IV co-injection of scAAV9-PGK-SMNopti in P0 SMNdelta7 mice

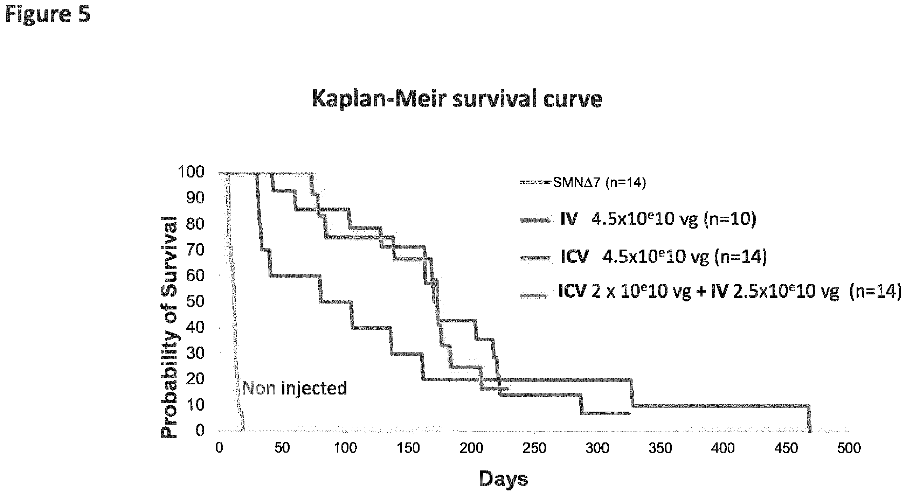

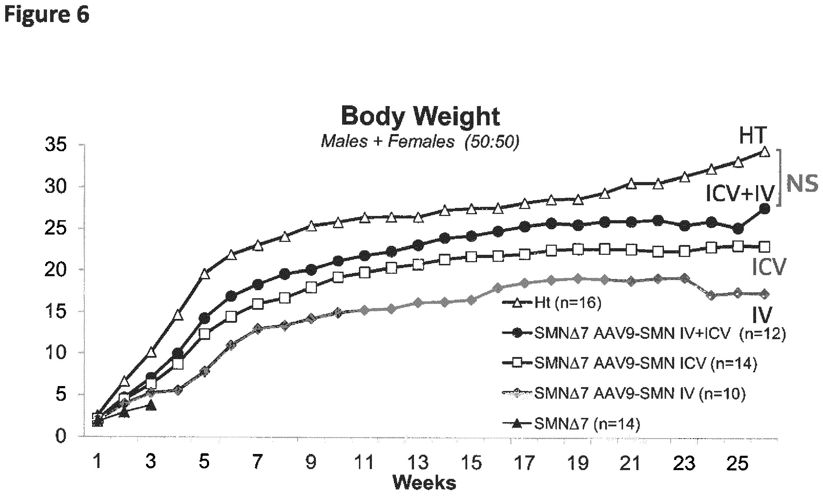

100% of the ICV / IV injected mice are still alive at 50 days post-injection (40% after IV (**Fig.5**). However, it is too early yet to detect any difference in survival between the ICV and IV/ICV groups.
- **Body weight of SMNdelta7 mice after ICV / IV co-injection of scAAV9-PGK-SMNopti in P0 SMNdelta7 mice.** The body weight loss phenotype of ICV/IV co-injected SMNdelta7 mice was improved compared to that of IV or ICV injected SMNdelta7 mice (Ht: control heterozygous mice; NI: non injected SMNdelta7 mice (**Fig. 6**).

### • Survival percentage after ICV / IV co-injection of scAAV9-PGK-SMNopti in P0 severe SMA mice

The mean survival of AAV9-SMN injected mice was increased compared to that of non-injected mice (3.7 ± 0.4, n=14), either after ICV (8.1 ± 2, n=7) or IV (9 ± 2.7, n=4) injection (**Fig. 7**). However, the median lifespan of IV/ICV mice (17 days) was superior to that of mice injected IV (12 days) or ICV (9 days) alone. The therapeutic effect of the combined IV/ICV injection was variable (7 to more than 60 days) due to interindividual variability of the severe SMA mice (Mean survival of the ICV 27 ± 13 days). One mouse out of 4 is still alive (at more than 60 days post-injection) in the combined IV/ICV group.

In addition, the weight body loss phenotype of ICV/IV co-injected KO mice is improved compared to that of IV or ICV injected SMNdelta7 mice (Hz: control Heterozygous mice; KO: non injected severe SMA mice; WT: wild type mice) (**Fig. 8**).

### Comparison of SMN expression in the CNS of ICV, IV, or Co-IV/ICV scAAV9-PGK-SMN injected SMNdelta7 mice

**Fig9** shows the SMN levels are reduced in Co-ICV/IV injected mice compared to mice injected with ICV alone. The number of SMN-positive cells and the intensity of the staining are also reduced in Co-ICV/IV injected mice compared to mice injected with ICV alone.

### Restoration of the reproductive capacity in treated SMA mice.

A significant benefit was observed in the SMNdelta7 female mice after ICV or ICV/IV injection in terms of restored reproductive capacity compared to IV injected mice (**Fig10**). 100% of the females were fertile in the ICV/IV and ICV treated groups, in contrast to IV treated mice which were not able to reproduce themselves.

### Comparison of the survival and body weight phenotype of wild-type mice and Co-ICV/IV AAV10-hSMNopti-injected hSMN2 mice.

The body weight loss phenotype and survival of Co-ICV/IV AAV10 injected mice was considerably improved compared to non-treated mice, in particular in two mice aged of 209 and 36 days (**Fig11**). Non-treated mice never passed the age of 6 days (median survival of ≈ 3 days).

### References

Azzouz, M., A. Hottinger, et al. (2000). "Increased motoneuron survival and improved neuromuscular function in transgenic ALS mice after intraspinal injection of an adeno-associated virus encoding Bcl-2." Hum Mol Genet 9(5): 803-11.
Azzouz, M, et al., (2004). " Lentivector-mediated SMN replacement in a mouse model of spinal muscular atrophy." J Clin Invest. 114(12):1726-31
Boillee, S., K. Yamanaka, et al. (2006). "Onset and progression in inherited ALS determined by motor neurons and microglia." Science 312(5778): 1389-92.
Cearley, C. N. and J. H. Wolfe (2006). "Transduction characteristics of adeno-associated virus vectors expressing cap serotypes 7, 8, 9, and Rh10 in the mouse brain." Mol Ther 13(3): 528-37.
Daly, T. M., C. Vogler, et al. (1999). "Neonatal gene transfer leads to widespread correction of pathology in a murine model of lysosomal storage disease." Proc Natl Acad Sci U S A 96(5): 2296-300.
Fu, H., J. Muenzer, et al. (2003). "Self-complementary adeno-associated virus serotype 2 vector: global distribution and broad dispersion of AAV-mediated transgene expression in mouse brain." Mol Ther 8(6): 911-7.
Fyfe, J. C., M. Menotti-Raymond, et al. (2006). "An approximately 140-kb deletion associated with feline spinal muscular atrophy implies an essential LIX1 function for motor neuron survival." Genome Res 16(9): 1084-90.
Inagaki, K., S. Fuess, et al. (2006). "Robust systemic transduction with AAV9 vectors in mice: efficient global cardiac gene transfer superior to that of AAV8." Mol Ther 14(1): 45-53.
Kaspar, BK, et al. (2003). "Retrograde viral delivery of IGF-1 prolongs survival in a mouse ALS model." Science 301: 839-42.
MacLean, H. E., G. L. Warne, et al. (1996). "Spinal and bulbar muscular atrophy: androgen receptor dysfunction caused by a trinucleotide repeat expansion." J Neurol Sci 135(2): 149-57.
McCarty, D. M., H. Fu, et al. (2003). "Adeno-associated virus terminal repeat (TR) mutant generates self-complementary vectors to overcome the rate-limiting step to transduction in vivo." Gene Ther 10(26): 2112-8.
Monani, U. R. (2005). "Spinal muscular atrophy: a deficiency in a ubiquitous protein; a motor neuron-specific disease." Neuron 48(6): 885-96.
Pasinelli, P. and R. H. Brown (2006). "Molecular biology of amyotrophic lateral sclerosis: insights from genetics." Nat Rev Neurosci 7(9): 710-23.
Passini, M. A. and J. H. Wolfe (2001). "Widespread gene delivery and structure-specific patterns of expression in the brain after intraventricular injections of neonatal mice with an adeno-associated virus vector." J Virol 75(24): 12382-92.
Scherrmann, J. M. (2002). Drug delivery to brain via the blood-brain barrier. Vascul Pharmacol. 38: 349-54.
Xiao, X., J. Li, et al. (1998). "Production of high-titer recombinant adeno-associated virus vectors in the absence of helper adenovirus." J Virol 72(3): 2224-32.

## Claims

1. An AAV9 or AAV10 vector for use in a method for expressing a therapeutic gene in a mammal, comprising the combined administration into the cerebrospinal fluid and into the blood of said mammal of said vector comprising said gene.

2. An AAV9 or AAV10 vector for use in a method for treating a multi-systemic disease in a mammal by administration of a therapeutic gene effective to treat said disease, wherein said therapeutic gene is comprised in said vector and wherein said method comprises the combined administration of the vector into the cerebrospinal fluid and into the blood of said mammal.

3. The vector for use according to claim 1 or 2, wherein the genome of the vector is single- or double-stranded.

4. The vector for use according to any one of claims 1 to 3, wherein the vector comprises a replication defective AAV genome lacking functional Rep and Cap coding viral sequences.

5. The vector for use according to any one of claims 1 to 4, wherein the vector is a ssAAV9 vector, an scAAV9 vector, or an AAV10 vector.

6. The vector for use according to claim 1 or 2, wherein administering the vector in the cerebrospinal fluid (CSF) of the mammal is performed by i.c.v. injection (ICV), intrathecal injection, or intra-cisterna magna injection.

7. The vector for use according to claim 8, which comprises administering the vector at least into one cerebral lateral ventricle.

8. The vector for use according to claim 1 or 2, wherein administering the vector in the blood of the mammal is performed by intravenous injection (IV), intramuscular injection, intraarterial injection, intraperitoneal injection, or subcutaneous injection.

9. The vector for use according to claim 8, which comprises an intravenous injection of the vector.

10. The vector for use according to claim 1 or 2, which comprises the combined ICV and IV injection of the vector.

11. The vector for use according to claim 1 or 2, wherein the combined administration comprises the administration in the CSF and in the blood of said mammal within less than 72 hours from each other, preferably within less than 48 hours, more preferably within less than 24 hours, further more preferably within less than 1 hour from each other.

12. The vector for use according to claim 11, wherein the combined administration is a substantially simultaneous injection.

13. The vector for use according to claim 1 or 2, wherein the ratio: dose administered in the CSF/dose administered in the blood is comprised between 0.2 and 5, preferably between 0.2 and 1.5.

14. The vector for use according to claim 13, wherein said ratio is 0.4, 0.6, 0.8, 1.0, or 1.25.

15. The vector for use according to of any one of the preceding claims, wherein the therapeutic gene encodes a therapeutic RNA or protein selected from growth factors, cytokines, hormones, neurotransmitters, enzymes, anti-apoptotic factors, angiogenic factors, any protein known to be mutated in pathological disorders such as the "survival of motor neuron" protein (SMN).

16. The vector for use according to any one of claims 2 to 17, wherein the multi-systemic disease is selected from neurodegenerative diseases, neuromuscular diseases, pain, lysosomal diseases, trauma, bone marrow diseases, cancers of the nervous system, demyelinating diseases, autoimmune diseases of the nervous system, neurotoxic syndromes, sleeping disorders.

17. The vector for use according to any one of the preceding claims, which comprises a single combined CSF-Blood administration.

18. An AAV9 or AAV10 vector for use in a method of treating SMA in a mammal in need thereof, comprising the combined administration into the cerebrospinal fluid and blood of said mammal of said vector comprising a SMN gene, said combined administration leading to expression of SMN protein in nervous system and peripheral tissues and organs and allowing the treatment of SMA.

19. A kit comprising two unitary dosages of an AAV9 or AAV10 vector comprising a therapeutic gene, one unitary dosage being adapted for systemic injection, and one unitary dosage being adapted for injection into the CSF.
